## Europäisches Patentamt
## European Patent Office
## Office européen des brevets

(19)

(11) Veröffentlichungsnummer: **0 241 701**
B1

(12)

# EUROPÄISCHE PATENTSCHRIFT

(45) Veröffentlichungstag der Patentschrift:
04.07.90

(51) Int. Cl.⁵: **A61G 13/00**, A61B 6/04

(21) Anmeldenummer: **87103359.3**

(22) Anmeldetag: **09.03.87**

(54) **Patientenlagerungstisch.**

(30) Priorität: **20.03.86 DE 8607732 U**

(43) Veröffentlichungstag der Anmeldung:
**21.10.87 Patentblatt 87/43**

(45) Bekanntmachung des Hinweises auf die Patenterteilung:
**04.07.90 Patentblatt 90/27**

(84) Benannte Vertragsstaaten:
**DE FR**

(56) Entgegenhaltungen:
**EP-A- 0 132 179**
**US-A- 1 656 760**
**US-A- 4 333 637**

(73) Patentinhaber: **Siemens Aktiengesellschaft,
Wittelsbacherplatz 2, D-8000 München 2(DE)**

(72) Erfinder: **Schäfer, Willi, Dipl.-Ing. (FH),
Genglerstrasse 20, D-8520 Erlangen(DE)**
Erfinder: **Uebelacker, Karl, Marquardsenstrasse 5,
D-8520 Erlangen(DE)**

ACTORUM AG

## Beschreibung

Die Erfindung betrifft einen Patientenlagerungstisch mit einer einseitig freitragend an dem Oberteil eines höhenverstellbaren Sockels lösbar gelagerten Tischplatte.

Es ist ein Patientenlagerungstisch bekannt, bei dem eine aus Kohlefasermaterial bestehende, dünne Tischplatte freitragend an einem Sockel gelagert ist, welcher an einer Säule an der Decke des Untersuchungsraumes aufgehängt ist. Dadurch ist ein guter Zugang zum Patienten möglich. Die Verbindung der Tischplatte mit der Decke des Untersuchungsraumes über eine Säule bedeutet jedoch einen großen konstruktiven Aufwand. Ferner ist die Decke nicht immer zum Aufhängen eines Patientenlagerungstisches geeignet.

In der EP-A 0 132 179 ist ein Patientenlagerungstisch mit einer einseitig freitragend am Oberteil eines höhenverstellbaren Sockels lösbar gelagerten Tischplatte beschrieben, bei dem das Oberteil mit einer Teleskopsäule um eine horizontale Achse schwenkbar verbunden ist. Durch das Schwenken des Oberteiles mit der Tischplatte können dabei unterschiedliche Neigungen der Tischplatte eingestellt werden. Das Innere der Teleskopsäule wird jedoch beim Schwenken des Oberteiles nicht freigelegt.

Der Erfindung liegt die Aufgabe zugrunde, einen Patientenlagerungstisch der eingangs genannten Art mit einem auf dem Fußboden stehenden Sockel so auszubilden, daß ein leichter Zugang zu den Teilen im Inneren des Sockels möglich ist.

Diese Aufgabe ist erfindungsgemäß dadurch gelöst, daß das Oberteil mit einer Teleskopsäule um eine horizontale Achse derart schwenkbar verbunden ist, daß das Innere der Teleskopsäule bei nach oben verschwenktem Oberteil zugänglich ist. Der Mechanismus für die Höhenverstellung liegt dabei im Inneren der Teleskopsäule. Er ist für Wartungszwecke leicht durch Aufklappen des Oberteiles zugänglich.

Die Erfindung ist nachfolgend anhand eines in der Zeichnung dargestellten Ausführungsbeispieles näher erläutert. Es zeigen:

Fig. 1 einen Patientenlagerungstisch nach der Erfindung, und

Fig. 2 einen Teil des Patientenlagerungstisches gemäß Figur 1 zur Erläuterung des Erfindungsgedankens.

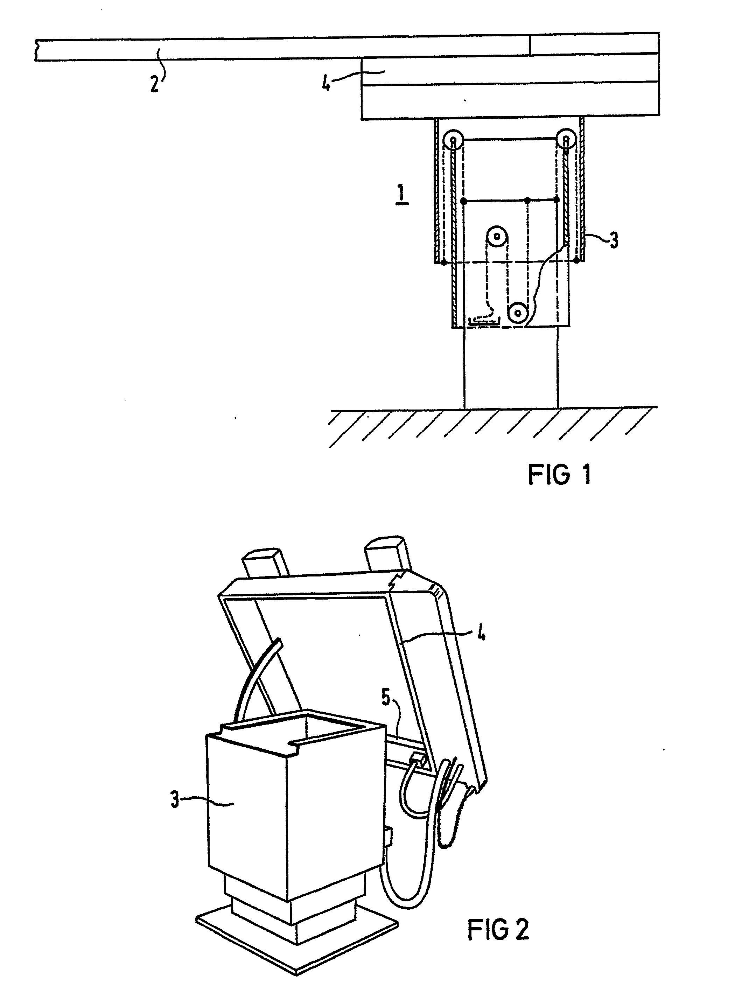

In der Figur 1 ist ein Sockel 1 dargestellt, auf dem eine Tischplatte 2 einseitig freitragend gehaltert ist. Der Sockel 1 weist eine Teleskopsäule 3 auf, welche über einen Kettenzug motorisch aus- und einfahrbar ist. Die Tischplatte 2 ist mit der Teleskopsäule 3 durch ein Oberteil 4 verbunden. Sie kann vom Oberteil 4 für Servicezwecke und zum Auswechseln gelöst werden.

Die Figur 2 zeigt die Komponenten 3 und 4 ohne die Tischplatte 2. Aus der Figur 2 geht hervor, daß das Oberteil 4 mit der Teleskopsäule 3 um eine nahe dem Fußende des Patientenlagerungstisches liegende Achse 5 schwenkbar verbunden ist. Für Servicezwecke kann demgemäß die Tischplatte 2 abgenommen und das Oberteil 4 in die in der Figur 2 dargestellte Lage verschwenkt werden, so daß das Innere der Teleskopsäule 3 leicht zugänglich ist. Es sind also in einfacher Weise Wartungsarbeiten im Inneren der Teleskopsäule 3, z.B. am Höhenverstellmechanismus des Patientenlagerungstisches, möglich, ohne daß hierzu ein Aufwendiges Entfernen von Verkleidungen erforderlich ist.

## Patentansprüche

Patientenlagerungstisch mit einer einseitig freitragend am Oberteil (4) eines höhenverstellbaren Sockels (1) lösbar gelagerten Tischplatte (2), bei dem das Oberteil (4) mit einer Teleskopsäule (3) um eine nahe einer Tischplattenkante liegende horizontale Achse (5) derart schwenkbar verbunden ist, daß das Innere der Teleskopsäule (3) bei nach oben verschwenktem Oberteil (4) zugänglich ist.

## Claims

Patient supporting table with a table top (2) which is detachable supported at one side in cantilevered fashion on the upper part (4) of a height-adjustable pedestal (1), in which the upper part (4) is connected to a telescopic column (3) so that this upper part (4) is pivotable around a horizontal axis (5) lying close to an edge of the table top, and in such a way that the interior of the telescopic column (3) is accessible when the upper part (4) is pivoted upwards.

## Revendications

Table formant couchette pour patient comportant un plateau (2) monté de façon amovible, en étant en porte-à-faux d'un côte, sur la partie supérieure (4) d'un socle (1) réglable en hauteur, et dans laquelle la partie supérieure (4) est reliée à une colonne télescopique (3) de manière à pouvoir pivoter autour d'un axe horizontal (5), situé à proximité d'un bord du plateau, de sorte que l'intérieur de la colonne télescopique (3) est accessible lorsque la partie supérieure (4) est pivotée vers le haut.

FIG 1

FIG 2